(19) **Europäisches Patentamt / European Patent Office / Office européen des brevets**

(11) **EP 3 064 594 A1**

(12) # EUROPEAN PATENT APPLICATION

(43) Date of publication:
07.09.2016 Bulletin 2016/36

(51) Int Cl.:
*C12Q 1/68* (2006.01)

(21) Application number: 15305347.5

(22) Date of filing: 06.03.2015

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(71) Applicants:
• **Université de Bordeaux**
  **33000 Bordeaux (FR)**
• **INSERM (Institut National de la Santé et de la Recherche Médicale)**
  **75013 Paris (FR)**

• **Institut Bergonié**
  **33076 Bordeaux (FR)**

(72) Inventors:
• **Chibon, Frédéric**
  **33160 Cestas (FR)**
• **Croce, Sabrina**
  **33140 Villenave d'Ornon (FR)**

(74) Representative: **Novagraaf Technologies**
**Bâtiment O2**
**2, rue Sarah Bernhardt**
**CS90017**
**92665 Asnières-sur-Seine Cedex (FR)**

(54) **Signature of clinical outcome in uterine smooth muscle tumor analysis, and method of diagnosis thereof**

(57) The present invention relates to a method for *in vitro* classify and/or distinguish, in a patient-derived biological sample of STUMP, truly malignant tumors from benign lesions within a biological sample belonging to the category of STUMP, especially those with equivocal morphological features, characterized in that it comprises the calculation of the Genomic Index (GI), i.e. the number and type of alterations of the GIST genome, according to the formula as follows:

$$GI = A^2/C,$$

wherein A is the total number of alterations in STUMP genome, consisting of segmental gains and losses, and C is the number of involved chromosomes,
and wherein said STUMP is classified in a group without recurrence or metastasis when the GI is less than 10, or in a group of tumors with significant recurrence when GI is equal or higher than 10.

The present invention also relates to a method for screening for compounds for the use in the treatment of STUMP, and to a method of follow-up of efficacy of a treatment of STUMP by a compound.

A

**STUMP - Genomic Index**

a Gl<10 : 15 cases
b Gl>10 : 13 cases

| | | 0 | 1 y | 2 y | 3 y | 4 y | 5 y |
|---|---|---|---|---|---|---|---|
| Gl<10 | Patients at risk | 15 | 14 | 14 | 11 | 11 | 10 |
| | Cumulated events | 0 | 0 | 0 | 0 | 0 | 0 |
| | Metastasis FS | 1 | 1 | 1 | 1 | 1 | 1 |
| Gl>10 | Patients at risk | 13 | 11 | 8 | 7 | 4 | 2 |
| | Cumulated events | 0 | 2 | 4 | 5 | 6 | 8 |
| | Metastasis FS | 1 | 0.85 | 0.69 | 0.61 | 0.52 | 0.26 |

**Figure 4 (1/3)**

## Description

### Technical field

The present invention refers to a method for in vitro classify and/or distinguish, in a patient-derived biological sample of STUMP, truly malignant tumors from benign lesions within a biological sample belonging to the category of STUMP, especially those with equivocal morphological features.

Therefore, the present invention has utility in the medical and pharmaceutical fields, especially in the field of diagnosis.

In the description below, the references into brackets ([ ]) refer to the listing of references situated at the end of the text.

### Background of the Invention

Smooth muscle uterine tumors include benign tumors- termed leiomyoma - that represent the most common tumor in women, and malignant tumors- such as leiomyosarcoma - that represent 1-2% of all uterine cancers and 40% of uterine sarcomas (Abeler VM et al. "Uterine sarcomas in Norway. A histopathological and prognostic survey of a total population from 1970 to 2000 including 419 patients". Histopathology 2009;54:355-64 ([1])). These two entities are differentiated based on the "three features" (cytological atypia, mitotic count and tumor cell necrosis) morphologic approach, first proposed by investigators from Stanford in 1994 (Bell SW et al. "Problematic uterine smooth muscle neoplasms. A clinicopathologic study of 213 cases". Am J Surg Pathol 1994;18:535-58 ([2])) and approved in 2014 by the WHO (Oliva E et al.: "Mesenchymal tumours. Smooth muscle tumour of uncertain malignant potential". In: Kurman RJ, Carcangiu ML, Herrington CS, Young RH, editors. WHO Classification of Tumours of Female Reproductive Organs.Lyon: IARC; 2014. p. 135-47 ([3])). While these guidelines allow to successfully classify most conventional leiomyomas and leiomyosarcomas, some problematic lesions do not completely fulfill the criteria for malignant tumors (eg diffuse moderate atypia but less than 10 mitotic figures in 10 high-power fields (hpf), or equivocal necrosis with less than 10 mitotic figures in10 HPF and without atypia, or a borderline mitotic count 8-10 mitotic figures in10 HPF associated with diffuse atypia without necrosis) and are difficult to classify.

Furthermore, in certain cases, a degree of subjectivity in the interpretation of these "three features" criteria, and the interposition of external factors such as prior treatments (hormonal therapy or embolization before surgery), or a non-optimal fixation of the specimen, may pose challenges that could lead to an equivocal diagnosis.

Some lesions with mild atypia and a benign appearance could, for instance, surprise with an unexpected recurrence or metastasis (Amant F et al.: "Report of an unusual problematic uterine smooth muscle neoplasm, emphasizing the prognostic importance of coagulative tumor cell necrosis". Int J Gynecol Cancer 2005;15:1210-2 ([18])). Some leiomyosarcomas cases with confirmed histological malignancy, on the other hand, could end up having a prolonged survival even if the overall prognosis for leiomyosarcomas is poor (D'Angelo E et al.: "Uterine leiomyosarcomas: tumor size, mitotic index, and biomarkers Ki67, and Bcl-2 identify two groups with different prognosis". Gynecol Oncol 2011;121:328-33 ([19]); Veras E et al.: "Low-grade leiomyosarcoma" and late-recurring smooth muscle tumors of the uterus: a heterogenous collection of frequently misdiagnosed tumors associated with an overall favorable prognosis relative to conventional uterine leiomyosarcomas". Am J Surg Pathol 2011;35:1626-37 ([20])).The pathologist's interpretation can also be particularly difficult and subjective, especially for patients who have ischemic, myxoid, or epithelioid modifications in leiomyoma caused by previously received endocrine therapy. Furthermore, an important inter-observer variability in the interpretation of tumor cell necrosis in smooth mesenchymal uterine lesions was recently reported, with complete disagreement being reported in 18% of the cases (Lim D et al.: "Interobserver variability in the interpretation of tumor cell necrosis in uterine leiomyosarcoma". Am J Surg Pathol 2013;37:650-8 ([21])). According to the 2014 WHO classification ([3]) "a smooth muscle tumor with features that preclude an unequivocal diagnosis of leiomyosarcoma, but that do not fulfill the criteria for leiomyoma, or its variants, and raise concern that the neoplasm may behave in a malignant fashion should be termed "smooth muscle tumor of uncertain malignant potential". Only the outcome will confirm its benign or malignant nature. A uterine smooth muscle tumor that cannot be unequivocally diagnosed as benign or malignant should be termed smooth muscle tumor with uncertain malignant potential (STUMP) ([3]).

Furthermore, STUMP represents a "managerial" category rather than a biological and clinical entity, and for this reason the use of this diagnostic term should be limited whenever possible. This borderline category for which malignancy cannot be confirmed and the prognosis cannot be determined represents a major diagnostic challenge that could have important clinical consequences for the treatment, as well as a profound human and social effect for the patient.

In order to complement the morphologic analysis in difficult cases, a variety of ancillary laboratory techniques (p53, p16, bcl2, ki67) have been implemented but without significant improvement in the diagnosis (de Vos S et al.: "p53 alterations in uterine leiomyosarcomas versus leiomyomas". Gynecol Oncol 1994;54:205-8 ([4]); Hall KL et al.: "Analysis of Ki-ras, p53, and MDM2 genes in uterine leiomyomas and leiomyosarcomas". Gynecol Oncol 1997;65:330-5 ([5]);.Jeffers MD et al.: "p53 immunoreactivity and mutation of the p53 gene in smooth muscle tumours of the uterine corpus". J Pathol 1995;177:65-70 **([6]);** Mayerhofer K et al.: "Ki-67 expression in patients with uterine leiomyomas, uterine smooth muscle tumors of uncertain malignant potential (STUMP) and uterine leiomyosarcomas (LMS)". Acta Obstet Gynecol Scand 2004;83:1085-8 **([7]);** Niemann TH et al.: "p53 protein overexpression in smooth muscle tumors of the uterus". Hum Pathol 1995;26:375-9 **([8]);** O'Neill CJ et al.: "Uterine leiomyosarcomas are characterized by high p16, p53 and MIB1 expression in comparison with usual leiomyomas, leiomyoma variants and smooth muscle tumours of uncertain malignant potential". Histopathology 2007;50:851-8 **([9])).**

By trying to avoid the diagnosis of STUMP, the pathologist could risk under- or over diagnosing a difficult lesion with the inherent risk of under or overtreatment, which could have dramatic consequences for the patient, especially in her reproductive age (hysterectomy precluding fertility).

Only a few reports have been published on STUMP (Ip PP et al.: "Uterine smooth muscle tumors of uncertain malignant potential (STUMP): a clinicopathologic analysis of 16 cases". Am J Surg Pathol 2009;33:992-1005 **([10]);** Ip PP et al.:" Uterine smooth muscle tumors other than the ordinary leiomyomas and leiomyosarcomas: a review of selected variants with emphasis on recent advances and unusual morphology that may cause concern for malignancy". Adv Anat Pathol 2010;17:91-11210 **([11]);**Ip PP, Cheung AN.: "Pathology of uterine leiomyosarcomas and smooth muscle tumours of uncertain malignant potential". Best Pract Res Clin Obstet Gynaecol 2011;25:691-704 **([12])),** and similarly, studies on the genomic profile of leiomyomas and leiomyosarcomas are relatively scarce and cover a very limited number of cases (Cho YL, Bae S, Koo MS, et al. Array comparative genomic hybridization analysis of uterine leiomyosarcoma. Gynecol Oncol 2005;99:545-51 **([13]);** Meadows KL et al.: "Genome-wide analysis of loss of heterozygosity and copy number amplification in uterine leiomyomas using the 100K single nucleotide polymorphism array". Exp Mol Pathol 2011;91:434-9 **([14]);** Raish M, Khurshid M, Ansari MA, et al. Analysis of molecular cytogenetic alterations in uterine leiomyosarcoma by array-based comparative genomic hybridization. J Cancer Res Clin Oncol 2012;138:1173-86 **([15])).** In the genomic era, few studies in the literature have reported on leiomyomas, leiomyosarcomas and array-CGH, and only for a limited number of cases. In 2005 Cho et al. **([13])** studied the genomic profile of four leiomyoma and seven leiomyosarcomas by array-CGH with 1440 human BACs. While uterine leiomyomas, did not present genomic alterations, all leiomyosarcomas showed the gains of 7q and 12q and several chromosome region losses. Although this study highlights the genomic differences between leiomyomas and leiomyosarcomas, supporting our hypothesis, the authors did not carry out the genome profiling as a practical tool to improve morphological diagnosis. Meadows et al. have recently studied loss of heterozygosity and copy number amplifications by high-density SNP platform (Affymetrix 100K) in 37 leiomyomas **([14]).** They observed that genome-wide loss of heterozygosity and copy number alterations are infrequent events in leiomyomas that generally do not have a clinical impact, confirming our hypothesis. They did not compare the leiomyomas with the leiomyosarcomas profile. Lastly, Raish et al. described the main genetic alterations in 15 leiomyosarcomas **([15]).**

Recently, a signature associated with instability and chromosomal complexity predictive of metastatic relapse in soft tissue sarcoma was published (Chibon F et al.: "Validated prediction of clinical outcome in sarcomas and multiple types of cancer on the basis of a gene expression signature related to genome complexity". Nat Med 2010;16:781-7 **([16])).** It has been demonstrated that this signature, based on expression profiles (CINSARC for Complexity INdex in SARComa, a 67 genes-expression prognostic signature related to genome complexity in sarcomas, WO/2010/122243 **([23]))** was closely linked to the level of rearrangement of the tumoral genome in GIST (Gastrointestinal Stromal Tumor), and created a way to identify a genomic signature combining the number and the type of tumoral genomic alterations evaluated by array-CGH (Array comparative genomic hybridization). The genomic signature (genomic index or GI) is a powerful predictor of metastatic relapse in GISTs, as is the CINSARC signature (WO2012/52954 **([24])).** Further, the array-CGH technique is adapted to formalin-fixed paraffin-embedded tumor material and its efficacy has been demonstrated on tumor samples that have been archived for over 15 years. However, none of these technics have been used in relation with STUMP, especially to distinguish a malignant tumor from a benign tumor.

The comparison of different studies on the clinical behavior of STUMP is difficult because of differing and evolving diagnostic criteria and the variable terminology, as often in literature "atypical leiomyomas" was employed as a synonym of STUMP as well as Bizarre Nuclei leiomyomas **([12])),** a benign variant of leiomyomas (Croce S. et al.: "Uterine Leiomyomas with Bizarre Nuclei: A Clinicopathologic Study of 59 cases". Am J Surg Pathol 2014 **([22])).** Elsewhere, the investigators from Stanford did not use the term "STUMP", but instead four categories of uterine smooth muscle tumors that had a low or uncertain malignant potential: atypical leiomyomas with limited experience, smooth muscle tumor with low malignant potential, atypical leiomyomas with low risk of recurrence and mitotically active leiomyomas with limited experience **([2]).** A meta-analysis performed on all published studies that can be grouped into the similar diagnostic criteria, with sufficient follow-up and pathologic details, reveals a recurrence rate ranging from 10.4% to 26.7% **([3]).** Regardless of the "virtual" category of STUMP, the genomic profile analysis is a pertinent and useful tool assisting the diagnosis in challenging smooth muscle uterine lesions.

In contrast to "high-grade" leiomyosarcomas, recurrent STUMP are in general biologically low grade tumors, as the recurrence is often delayed and the clinical course prolonged **([12]).**

In view of all these elements, it clearly still exists a need of new tools allowing to predict outcome of STUMP, notably to distinguish malignant STUMP from benign STUMP, and palliating the failures, drawbacks and obstacles of the state of the art.

**Description of the invention**

After important researches on series of problematic uterine smooth muscle lesions, the Applicant found surprisingly that genomic profile analysis, notably by array-CGH, could split the group of borderline cases (i.e. STUMP) into two main groups consisting of (1) benign tumors (leiomyomas) with a low number of chromosomal alterations and (2) malignant ones (leiomyosarcomas) with a high chromosomal instability.

The Applicant surprisingly found that the clinical behavior of smooth muscle uterine tumors is strongly associated with specific genomic profiles.

In their study, the Applicant analyzed by array-CGH the genomic profile of a series of 29 problematic uterine smooth muscle lesions for which the assessment of the mitotic count, or of the presence and the degree of atypia, or of the nature of the necrosis were previously not conclusive and for which there previously was a poor inter-observer agreement.

To test the pertinence of the genomic profile analysis, the Applicant first proceeded to the study of benign (leiomyomas) and malignant lesions (leiomyosarcomas) for which there previously was a complete diagnostic agreement. The Applicant surprisingly observed a perfect concordance between morphology, clinical behavior and genomic index (GI). Genomic index was then applied to split the series of problematic smooth muscle uterine tumors into two groups: the first one (genomic index<10) without recurrence or metastasis and similar behavior to that of leiomyoma; and the second group (genomic index> 10) consisting of tumors with significant recurrence due to disease that encompasses a spectrum of smooth muscle lesions approaching the recurrence risk level of leiomyosarcomas but a more indolent, less aggressive course compared to leiomyosarcomas.

The Applicant surprisingly showed that, among the morphologic parameters of Stanford criteria ([3]):

-   the atypia is closest to GI as prognostic prediction, even if the presence of atypia alone does not allow the diagnosis of leiomyosarcomas,
-   mitotic activity is not a good single diagnostic parameter since it could reflect a reactive process. Furthermore, the mitotic count could be difficult to assess, especially in the ischemic areas where smudged nuclei in cellular degeneration or apoptosis may be confused with true mitotic figures causing an important inter-observer variability.

As described above, the genomic index divides problematic uterine smooth muscle lesions into leiomyomas and leiomyosarcomas. However, this enlarges the range of leiomyosarcomas adding more indolent cases to leiomyosarcomas, which conventionally have a more aggressive clinical course. In other words, the Applicant has demonstrated that genomic index is a powerful tool that allows splitting the problematic uterine smooth muscle lesions in two categories according to the risk of recurrence: a group of benign tumors, similar to leiomyomas, and a group of malignant lesions, similar to leiomyosarcomas. Therefore, the genomic analysis by array-CGH is an innovative diagnostic tool for smooth muscle uterine tumors, complementary to the morphological approach, and it is suitable for differentiating between benign (leiomyomas) and malignant tumors (leiomyosarcomas) and for providing an improved classification of the intermediate category with uncertain malignancy (STUMP) in cases with equivocal morphological features.

This is the first report on the use of genomic profile analysis by array-CGH as a diagnostic tool and of a genomic index threshold (<10 and >10) as a criterion for malignancy.

The Applicant surprisingly managed to construct a decisional algorithm based on GI.

The Applicant also surprisingly found that the genomic index is an independent prognostic marker in uterine mesenchymal lesions in terms of metastasis-free survival. The non-significant results in term of overall survival could be explained by the fact that there are more metastatic recurrence events in the cohort than deaths.

Accordingly, in a first aspect, the invention provides a method for *in vitro* classify and/or distinguish, in a patient-derived biological sample of STUMP, truly malignant tumors from benign lesions, especially those with equivocal morphological features, characterized in that it comprises the calculation of the Genomic Index (GI), i.e. the number and type of alterations of the GIST genome, according to the formula as follows:

$$GI = A^2 /C,$$

wherein A is the total number of alterations in STUMP genome, consisting of segmental gains and losses, and C is the number of involved chromosomes.

"STUMP" refers herein to any uterine smooth muscle tumor that cannot be unequivocally diagnosed as benign or malignant and should be termed smooth muscle tumor with uncertain malignant potential. In other words, it can be any problematic uterine smooth muscle lesions which do not completely fulfill the criteria for malignant tumors. For example, it can be any lesion presenting diffuse moderate atypia but less than 10 mitotic figures in 10 high-power fields (hpf), or equivocal necrosis with less than 10 mitotic figures/ in10 HPF and without atypia, or a borderline mitotic count 8-10 mitotic figures/ in10 HPF associated with diffuse atypia without necrosis) and which is difficult to classify.

"Patient-derived biological sample of STUMP" refers herein to any biological sample containing smooth muscle tumor of uncertain malignant potential from a patient treated by hysterectomy, or myomectomy, or biopsy performed, in a context of uterine mass.

"Number of alterations in STUMP genome" refers herein to different numerical and segmental gains and losses. The alterations may for example involve whole chromosome arms or chromosome without rearrangement, or intra-chromosome gains or losses. It may be measured by techniques known in the art, such as CGH-array (Comparative Genomic Hybridization array), or ExomeSequencing, and/or as taught by Lagarde et al. (Lagarde P et al. Mitotic checkpoints and chromosome instability are strong predictors of clinical outcome in gastrointestinal stromal tumors. Clin Cancer Res 2012;18:826-38 **([17]))**.

"Number of involved chromosomes" refers herein to the number of chromosomes of STUMP cells having an alteration. The number of chromosome may be measured by CGH-array

"Equivocal morphological features" refers herein to any lesion presenting diffuse moderate atypia but less than 10 mitotic figures in 10 high-power fields (hpf), or equivocal necrosis with less than 10 mitotic figures in10 HPF and without atypia, or a borderline mitotic count 8-10 mitotic figures in10 HPF associated with diffuse atypia without necrosis) which is difficult to classify.

Advantageously, STUMP is classified in a group without recurrence or metastasis when the GI is less than 10, or in a group of tumors with significant recurrence when GI is equal or higher than 10.

"Without recurrence or metastasis" refers herein to an absence of metastasis or disease during 5 years after STUMP outcome or after the end of a treatment of STUMP.

"Significant recurrence" refers herein to an increased risk to develop metastases or disease within 5 years after STUMP outcome or after the end of a treatment compared with a biological sample derived from a healthy person.

More particularly, the group without recurrence or metastasis may have a similar behavior to that of leiomyoma. "Similar behavior" refers herein to absence of local or distant recurrences.

Advantageously, in the group of tumors with significant recurrence, the recurrence may be due to disease that encompasses a spectrum of smooth muscle lesions approaching the recurrence risk level of leiomyosarcomas but a more indolent, less aggressive course compared to leiomyosarcomas.

In the method of the invention, the total number of alterations and the number of involved chromosomes in STUMP in the sample are determined by a genomic analysis by Array comparative genomic hybridization.

Another object of the invention relates to a method for in vitro predicting survival and/or clinical outcome of a biological sample belonging to the category of STUMP, characterized in that it comprises the calculation of the Genomic Index (GI), i.e. the number and type of alterations of the STUMP genome, according to the formula as follows:

$$GI = A^2 / C,$$

wherein A is the total number of alterations, consisting of segmental gains and losses, and C is the number of involved chromosomes in STUMP in the sample.

"Predicting survival and/or metastatic outcome" refers herein to predicting whether a patient has a chance to survive, or a risk to develop metastases following the outcome of a STUMP. The survival or development of metastases may be calculated from the date of initial diagnosis to the date of first metastases, relapse, last follow-up or death for patients with diagnosis of metastasis. According to a particular embodiment of the invention, STUMP may be classified in a group with high risk to develop metastases within 5 years of an outcome of STUMP, or in a group with no risk to develop metastases within 5 years, or in an intermediate group. More particularly, the group of patient with high risk to develop metastases within 5 years is characterized by a risk to develop metastases within 5 years of more than 80%, when GI is higher than 10, compared to a group with no risk to develop metastases within 5 years when GI is equal or less than 10.

Advantageously, the method of the invention allows classifying a STUMP in a group with low risk to develop metastases within 5 years, i.e. with a risk to develop metastases within 5 years equal to 0%, when GI is equal or less than 10.

Advantageously, the method of the invention allows classifying a STUMP in a group with increased risk to develop metastases within 5 years, when GI is more than 10.

Another object of the invention relates to a kit for the *in vitro* prediction of the survival outcome of a patient suffering from STUMP, and/or the development of metastases in a patient treated for or suffering from STUMP and/or the prediction of the efficacy of a treatment for STUMP, characterized in that it comprises means for the calculation of the GI.

"Means for the calculation of the GI" refers herein to any means allowing the calculation of the number of alterations in STUMP genome and of the number of involved chromosomes in STUMP. It can be any means known by the man skilled in the art, for example CGH-Array.

Another object of the invention relates to a method for screening compounds for the use in the treatment of STUMP, characterized in that it comprises the steps of:

 a. Contacting a test compound with a patient-derived biological sample containing STUMP cells,
 b. Calculating GI,
 c. Eventually comparing said GI with the GI before the contact between said test compound and said sample,
 d. Selecting said test compound allowing a down-regulation of the GI to 10 or less, wherein the STUMP is a leiomyosarcoma.

Another object of the invention relates to a method of follow-up of efficacy of a treatment of STUMP by a compound, characterized in that it comprises the steps of

 a. Calculating GI in a patient-derived biological sample containing STUMP cells before the beginning of the treatment of the patient by the compound,
 b. Calculating GI in a patient-derived biological sample containing STUMP cells during the the treatment of the patient by the compound,
 c. Comparing said GI of step a) and said GI of step b),
 wherein the decrease of the GI calculated in step b) indicates an efficient treatment of STUMP.

This invention is further illustrated by the following examples with regard to the annexed drawings.

**Brief description of the figures**

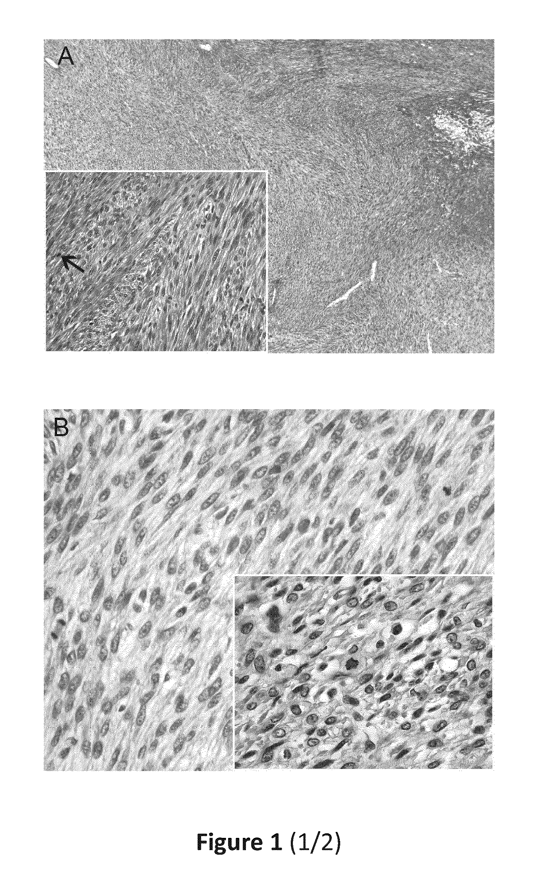

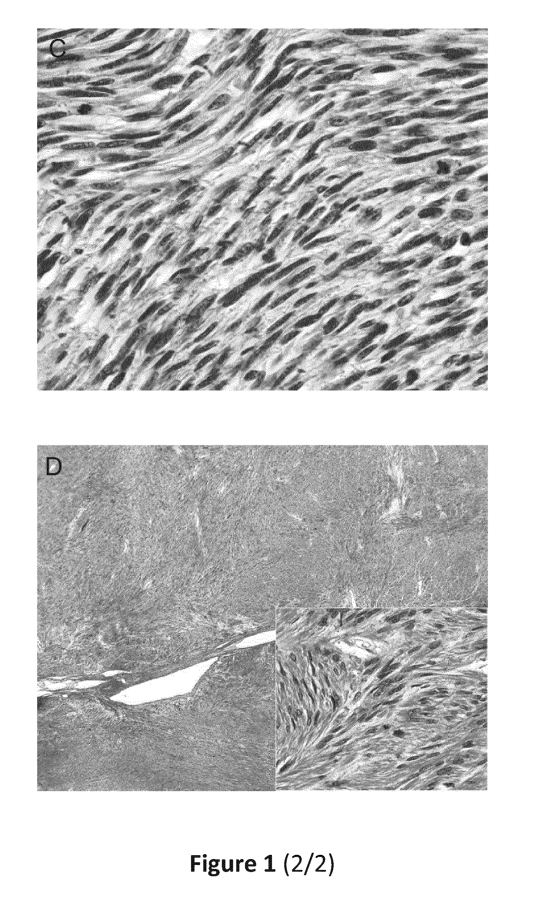

- Figure 1: represents the morphologic features of some challenging uterine smooth muscle tumors. **1A**. Case 17: cellular smooth muscle tumor with mild atypia and 20 mitotic figures (arrow). **1B**. This patient developed a pelvic and para-aortic recurrences 36 and 48 months later. The atypia is more prominent and mitotic count is higher. 1C. Case 21: highly cellular smooth muscle tumor composed of monotonous atypical spindle cells with brisk mitotic activity. The patient is alive with disease at 156 months after two recurrences. **1D**. Case 25: cellular spindle cell tumor with diffuse mild to moderate atypia and seven mitotic figures. The patient developed bone metastases 10 months after the diagnosis and died of disease 16 months later.

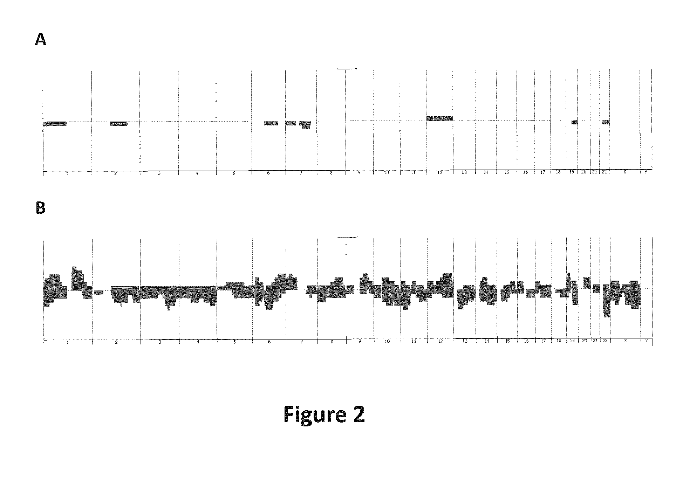

- Figure 2: represents penetrance plots corresponding to the genomic profiles of the ten leiomyomas **(2A)** and ten leiomyosarcomas **(2B)**. Chromosomes are presented from 1 to Y on the X axis and alteration frequencies on Y axis. Above and below areas compared to the middle axis represent losses and gains, respectively.

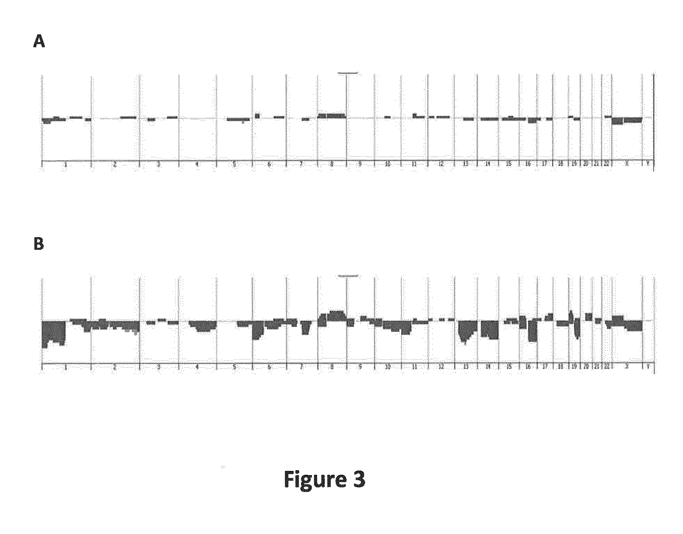

- Figure 3: represents penetrance plots corresponding to genomic profiles of the 16 STUMP with genomic index<10 **(3A)** and 13 STUMP with genomic index ≥10 **(3B)**.
 Chromosomes are presented from 1 to Y on the X axis and alteration frequencies on Y axis. Above and below areas compared to the middle axis represent losses and gains, respectively.

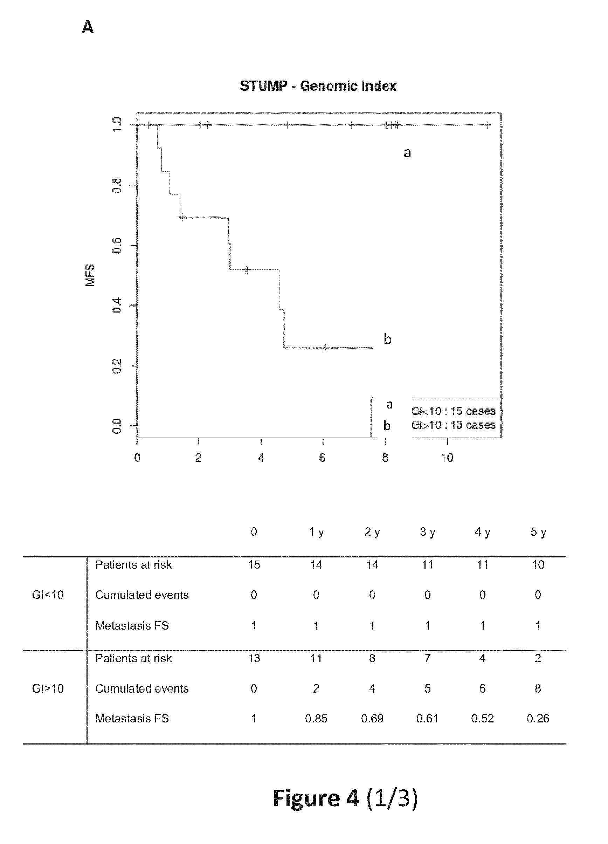

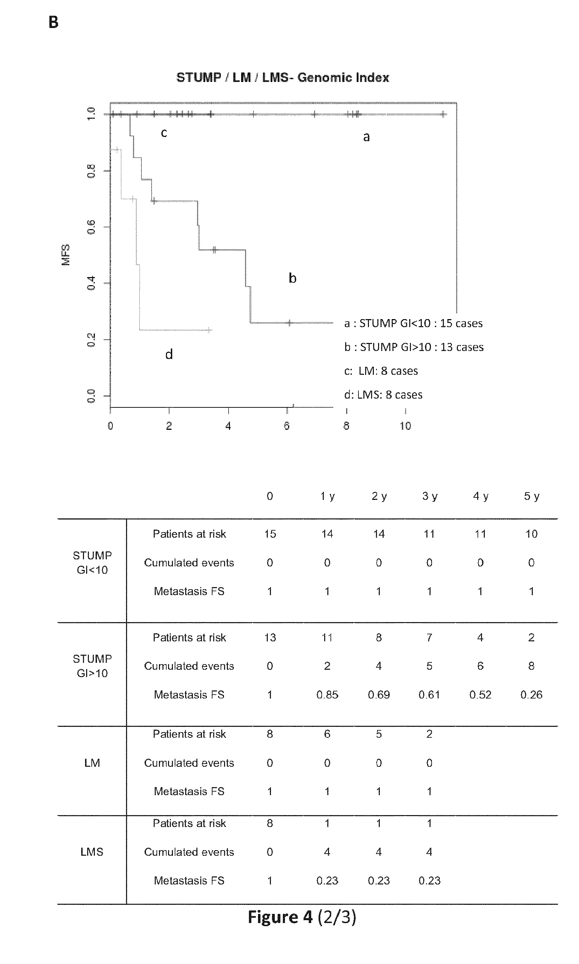

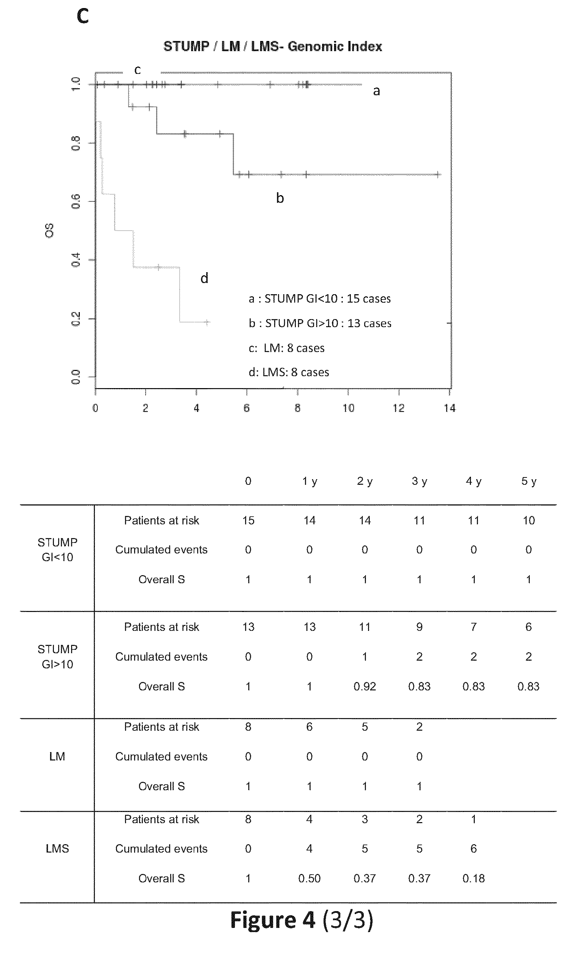

- Figure 4: represents Kaplan-Meier analysis of metastasis-free survival (MFS) for STUMP **(4A**, with GI<10 is represented by a curve and GI>10 is represented by b curve) and metastasis-free survival (MFS) for STUMP, leiomyomas and leiomyosarcomas **(4B** with GI<10 is represented by a curve, GI>10 is represented by b curve, LM is represented by c curve and LMS is represented by d curve) according to genomic index (GI).
 Kaplan-Meier analysis of overall survival **(4C)** of STUMP split according to genomic index (GI) compared with leiomyomas (LM) and LMS, with GI<10 is represented by a curve, GI>10 is represented by b curve, LM is represented by c curve and LMS is represented by d curve.

**Examples**

In all the examples and figures : **OS=** overall survival; **MFS=** metastasis-free survival; **GI1**= genomic index<10; **GI2**= genomic index ≥10.

**Example 1: Material and methods**

**Material and Methods**

**Tumor samples**

Formalin-fixed paraffin-embedded tumor tissues from 29 uterine smooth muscle lesions diagnosed as STUMP were obtained from the pathology archives of 12 Centres in France, Belgium and Czech Republic (Institut Bergonié of Bordeaux, Université libre de Bruxelles, Katholieke Universiteit of Leuven, University of Prague, Hôpital La Croix Rousse of Lyon, Centre Jean Perrin of Clermont Ferrand, Centre JF Leclerc of Dijon, Centre A Vautrin of Nancy, Centre Paul Papin of Angers, Centre Oscar Lambret of Lille, Institut Gustave Roussy of Villejuif and University Hospital of Montpellier). All cases were centrally reviewed by two gyneco-pathologists (SC and GMG). The presence of atypia, the mitotic count and the presence of tumor cell necrosis were assessed according to the Stanford criteria **([2]).**

The samples issued by the tumor archives of each department participating in the study were centralized in the Biological Resources Center of Institut Bergonié, which received the agreement from the French authorities to deliver samples for scientific research (AC-2008-812).

The control group used as a reference for the assessment of genomic index consisted of ten formalin-fixed paraffin-embedded conventional leiomyomas and ten conventional leiomyosarcomas with unquestionable features of benignity (for leiomyomas) and malignity (for leiomyosarcomas) that were collected from three of the participating institutions (Bruxelles, Bordeaux and Dijon).

**Array-comparative genomic hybridization analysis**

DNA was hybridized to 8x60K whole-genome Agilent arrays (G4450A; Agilent Technologies, Santa Clara, CA, USA) according to the manufacturer's protocol. The ADM-2 algorithm (Genomic Workbench, Agilent Technologies) of the CGH Analytics v4.0.76 software (Agilent Technologies) was used to identify DNA copy number anomalies at the probe level. A low-level copy number gain was defined as a log 2 ratio >0.25 and a copy number loss was defined as a log 2 ratio <0.25. A high-level gain or amplification was defined as a log 2 ratio > 1.5 and a homozygous deletion was suspected when the ratio was <-1.

The genomic index was calculated for each profile as follows: genomic index =$A^2/C$, where A is the total number of alterations (segmental gains and losses) and C is the number of involved chromosomes.

**Statistical Analysis**

Metastasis-free survival was calculated by the Kaplan-Meier method (Kaplan, E. L.; Meier, P.: Nonparametric estimation from incomplete observations. J. Amer. Statist. Assn. 53:457-481, 1958 ([25])) from the date of initial diagnosis to the date of first metastasis or last follow-up. Overall survival using the Kaplan-Meier method was calculated from the date of diagnosis to death or last follow-up. Survival curves were compared with the log-rank test. All survival analyses were performed using the R software version 2.14.1 and the "survival" package (Terry Therneau 2-02). A Package for Survival Analysis in S. R package version 2.36-14. Multivariate analysis was done using Cox regression with Firth's correction (R by Meinhard Ploner and Fortran by Georg Heinze 2011). coxphf: Cox regression with Firth's penalized likelihood. R package version 1.08. http://CRAN.R-project.org/package=coxphf).

**Results**

**Clinico-Pathologic and genomic data**

Clinico-pathologic data of the 29 samples are summarized in Table 1 for the STUMP series. Morphologically, all cases were uterine smooth muscle lesions of conventional (spindle cell) type, without any epithelioid or myxoid component (Figure 1). Regarding the diagnosis of STUMP, full agreement was obtained in 7/29 cases and 22/29 had discordant diagnoses. The cause of disagreement was the appreciation of the nature of the necrosis (tumoral versus ischemic necrosis) in 16/22 cases, and the extension (focal or diffuse) and the degree of the atypia (mild, moderate or high) in the remaining 6/22 cases. Among the latter, 1/6 tumor (case 2) presented marked nuclear atypia consisting of multinucleated giant cells in a patchy distribution with presence of interspersed normal cells and 6 mitotic figures/10 HPF, favoring a diagnosis of bizarre nuclei-leiomyomas. For the remaining 5/6 cases the degree of atypia was estimated high by the pathologist and low by the reviewers. For 3/8 patients with recurrent disease we could analyze the second tumor, whose morphology led to a clear categorization of the tumor as a leiomyosarcomas (Figure 1b). The average age was 50 years (range 24 to 85) and the median tumor size 7.7 cm. Twenty-two out of 29 patients underwent hysterectomy (for case 28 a curetting was followed by hysterectomy), 6/29 patients had myomectomy and for 1/29 patient (case 24) the surgical procedure was not specified. All tumors were stage I at presentation except one that was stage II (case 13) and another one (case 21) for which the status was unknown. For 27 tumors the quality of the surgery was optimal (R0), for one case suboptimal (R1) and for the remaining case the data was unknown. The follow-up period ranged from 12 to 156 months (overall average 70 months; 70 months in patients with myomectomy and 39 months for patients with hysterectomy) and details were obtained for all patients but one (case 7). For the leiomyomas control group follow-up details were obtained for 8/10 patients and all were alive and free of disease. Follow-up was available for 8/10 patients of the leiomyosarcomas control group (6/8 patients died due to disease and 2/8 were alive with disease).

**Table 1 Legend. STUMP:** smooth muscle lesion with uncertain malignant potential; **MA-LM:** mitotically active leiomyoma; **BN-LM:** bizarre nuclei leiomyoma; **NED:** not evidence of disease; **AWD:** alive with disease; **DOD:** dead of disease; **alive NOS:** alive not otherwise specified; **lost:** lost at follow up.

| Case | Age | GI | Tumoral necrosis | Mitoses | Atypia | DIAGNOSIS Before centralized review | DIAGNOSIS After centralized review | Location | Seize cm | Type and Quality of surgery | Recurrence / Metastasis | Recurrence (months) | 2° Recurrence (months) | Follow up (months) | Status |
|---|---|---|---|---|---|---|---|---|---|---|---|---|---|---|---|
| 1 | 32 | 0 | no | 1 | no | STUMP | LM | uterus | 6.5 | myomectomy R0 | no | | | 101 | NED |
| 2 | 44 | 0 | no | 6 | no | STUMP | BN-LM | uterus | 4.5 | hysterectomy R0 | no | | | 102 | NED |
| 3 | 67 | 0 | no | 2 | no | STUMP | LM | uterus | 1.5 | hysterectomy R0 | no | | | 102 | NED |
| 4 | 68 | 0 | no | 1 | no | STUMP | LM | uterus | 1.2 | hysterectomy R0 | no | | | 102 | NED |
| 5 | 46 | 0 | no | 5 | no | STUMP | LM | uterus | 6 | hysterectomy R0 | no | | | 24 | NED |
| 6 | 35 | 0 | no | 3 | no | STUMP | LM | uterus | 9 | myomectomy R0 | no | | | 24 | NED |
| 7 | 42 | 0 | no | 2 | no | STUMP | LM | uterus | 8 | hysterectomy R0 | no | | | | lost |
| 8 | 40 | 1 | no | 2 | no | STUMP | LM | uterus | 4.5 | hysterectomy R0 | no | | | 98 | NED |
| 9 | 36 | 1 | no | 13 | no | STUMP | MA-LM | uterus | 15 | myomectomy R0 | no | | | 137 | NED |
| 10 | 44 | 1 | no | 4 | no | STUMP | LM | uterus | 4.5 | hysterectomy R0 | no | | | 137 | NED |
| 11 | 41 | 2 | no | 1 | no | STUMP | LM | uterus | 8 | myomectomy R0 | no | | | 24 | NED |
| 12 | 24 | 2 | no | 7 | no | STUMP | cellular LM | uterus | 10 | myomectomy R0 | no | | | 12 | NED |
| 13 | 80 | 3 | no | 10 | no | STUMP | cellular LM | uterus+ pelvis | 15 | hysterectomy R1 | no | | | 59 | NED |

| Case | Age | GI | Tumoral necrosis | Mitoses | Atypia | DIAGNOSIS | | Location | Seize cm | Type and Quality of surgery | Recurrence / Metastasis | Recurrence (months) | 2° Recurrence (months) | Follow up (months) | Status |
|---|---|---|---|---|---|---|---|---|---|---|---|---|---|---|---|
| | | | | | | Before centralized review | After centralized review | | | | | | | | |
| 14 | 36 | 8.16 | no | 5 | yes | STUMP | STUMP | uterus | 7 | hysterectomy R0 | no | | | 84 | NED |
| 15 | 34 | 8.3 | no | 6 | no | STUMP | LM | uterus | ND | myomectomy R0 | no | | | 101 | NED |
| 16 | 47 | 9.14 | yes | 3 | no | STUMP | STUMP | uterus | 6 | hysterectomy R0 | no | | | 100 | NED |
| 17 | 46 | 11.6 | no | 20 | yes | STUMP | LMS | uterus | 5 | hysterectomy morcellation | parauterine, pre-cave LN | 36 | 12.4 | 69 | NED |
| 18 | 48 | 12 | no | 8 | yes | STUMP | STUMP | uterus | ND | hysterctomy R not known | soft tissues bone and pelvis | 13 | 76 | 89 | AWD |
| 19 | 43 | 13.1 | yes | 16 | no | STUMP | LMS | uterus | 8 | hysterectomy R0 | no | | | 43 | NED |
| 20 | 48 | 14.3 | no | 0 | no | STUMP | LM | uterus | 7 | hysterectomy R0 | no | | | 101 | NED |
| 21 | 48 | 16.3 | no | 20 | yes | STUMP | LMS | uterus | 7 | hysterectomy R0 | pelvis lung omentum paraaortic LN bladder rectum | 36 | 72 | 156 | AWD |
| 22 | 50 | 19.7 | no | 16 | yes | STUMP | STUMP | uterus | ND | hysterectomy R0 | paravertebral (soft tissues) | 58 | | 60 | AWD |
| 23 | 66 | 21.3 | yes | 14 | yes | STUMP | LMS | uterus | 8 | hysterectomy R0 | vagina | 56 | | 64 | DOD |
| 24 | 66 | 29.5 | no | 0 | no | STUMP | LM | uterus | ND | resection | peritoneum and pelvis | 8 | | 30 | DOD |

(continued)

| Case | Age | GI | Tumoral necrosis | Mitoses | Atypia | DIAGNOSIS | | Location | Seize cm | Type and Quality of surgery | Recurrence / Metastasis | Recurrence (months) | 2° Recurrence (months) | Follow up (months) | Status |
|---|---|---|---|---|---|---|---|---|---|---|---|---|---|---|---|
| | | | | | | Before centralized review | After centralized review | | | | | | | | |
| 25 | 43 | 45.6 | no | 7 | yes | STUMP | STUMP | uterus | 20 | hysterectomy R0 | bones | 10 | | 16 | DOD |
| 26 | 77 | 48 | no | 19 | yes | STUMP | LMS | uterus | 11 | hysterectomy R0 | ileum, vaginal top, fossa iliaca | 17 | | 25 | alive NOS |
| 27 | 85 | 60.2 | no | 0 | yes | STUMP | STUMP | uterus | 11 | hysterectomy R0 | no | | | 74 | NED |
| 28 | 61 | 60.3 | yes | 15 | no | STUMP | LMS | uterus | 2 | curettage + hysterectomy R0 | no | | | 34 | NED |
| 29 | 64 | 64.7 | no | 9 | yes | STUMP | STUMP | uterus | 7 | hysterectomy R0 | no | | | 18 | NED |

**Genomic data and clinical correlation**

The reference group of leiomyomas presented a flat genomic profile with no or only very few sporadic alterations (Figure 2a). The group of leiomyosarcomas, on the other hand, showed a rearranged chromosome profile with numerous intrachromosomal breaks (Figure 2b). Deletions occurred more often than gains, the most frequent ones being 22q (70%), 13q13-14 (50%), 11p14-15 (50%), 6q12-16 (50%), and 3q25 (50%) and 1q21-22 (60%), respectively (Figure 2c).

The two groups of tumors were therefore clearly distinct from each other as the genomic index assessment showed that all the leiomyomas harbored a genomic index <10 (between 0 and 7) and all leiomyosarcomas showed a genomic index >10 (between 19.6 and 80). The genomic study mirrored perfectly the morphologic analysis and stratified the tumors in a benign group (leiomyomas) with a genomic index <10, and in a malignant group (leiomyosarcomas), involving a significant rate of metastases and unfavorable outcome, and with a genomic index >10.

Among the patients of the leiomyomas group, 7/10 were alive without evidence of disease, one patient was alive with breast cancer and 2/10 were lost at follow-up. Among the patients with leiomyosarcomas, 6/10 were dead of disease, 2/10 alive with disease and 2/10 lost at follow-up.

Upon establishing the threshold for malignancy at genomic index=10, the genomic analysis was applied to STUMP tumors.

The STUMP analysis showed a more heterogeneous group of genomic profiles since we observed tumors with an even profile (Figure 3a), as well as those with a rearranged profile (Figure 3b). The latter harbored mainly the same alterations as leiomyosarcomas except for gains that were more frequent in leiomyosarcomas than in STUMP, and losses at 3q25, 6q12-16 and 22q that were less frequent in rearranged STUMP.

In 16/29 cases the genomic index was <10 (Table 1). For this group of tumors, the follow-up was available in 15/16 cases and all patients were alive without evidence of disease after an average time of 80 months (from 12 to 137 months). Two cases showing some worrisome morphologic features such as diffuse atypia (case 14) and tumor necrosis (case 16) displayed a relatively flat, "leiomyoma-type" benign profile in the genomic analysis. In this group of tumors no recurrence was recorded.

For the group of 13/29 tumors with genomic index>10, 3/13 patients were dead of disease at 16, 30 and 64 months after the diagnosis and the recurrences appeared at 8, 10 and 56 months from the first diagnosis. Three out of thirteen patients were alive with disease with a follow-up of 60, 89 and 156 months. For 2/3, the recurrences were multiple in time and location (Table 1). One patient was alive, not otherwise specified (with 25 months of follow-up) and 6/13 patients were alive without evidence of disease with an average time of 60 months (from 16 to 156 months). Of note, in the group of patients alive without evidence of disease with genomic index>10, one patient (case 17) suffered two recurrences: one at 36 months from diagnosis and the second 12.4 months after the first recurrence. The alive not otherwise specified patient also had a recurrence 17 months after the first diagnosis.

The stratification according to the genomic index=10 threshold splits the STUMP category of tumors into two groups with different outcomes and a p value not significant for overall survival and significant (p 0.00029, HR [95 % IC] 3.2.10e9 [0-inf]) for metastasis-free survival (Table 2, Figure 4a). When comparing leiomyoma, leiomyosarcomas and STUMP groups we observed that, whereas the STUMP with genomic index<10 showed the same clinical outcome as leiomyomas, those with genomic index>10 behaved like leiomyosarcomas (Figures 4b-c).

We tested the prognostic value of atypia and found that it was statistically significant for metastasis-free survival (p 0.00060, HR[IC 95%] 15.82[1.93-129.74]) but not for overall survival. The presence of mitosis (cut-off of 10) and necrosis were not significant for either overall survival or metastasis-free survival (Table2).

By multivariate analysis, genomic index was the only independent prognosis factor for metastasis-free survival (p 0.036, HR [IC 95%] 17.4 [1.17 - 2509]) (Table 2).

### Table 2: Prognostic value of Genomic Index in STUMP

| Univariate | | | | | Multivariate | | |
|---|---|---|---|---|---|---|---|
| | OS | | MFS | | MFS | | |
| | P | HR[95% CI] | P | HR [95% CI] | | P | HR [95% CI] |
| Genomic Index | NS | - | 0.00029 | 3.2.10e9 [0-INF] | Genomic Index | 0.036 | 17.4 [1.17 - 2509] |
| Atypia | NS | - | 0.00060 | 15.82 [1.93-129.74] | Atypia | NS | - |
| Mitoses | NS | - | NS | - | | | |
| Necrosis | NS | - | NS | - | | | |

**Reference List**

[1] Abeler VM, Royne O, Thoresen S, Danielsen HE, Nesland JM, Kristensen GB. Uterine sarcomas in Norway. A histopathological and prognostic survey of a total population from 1970 to 2000 including 419 patients. Histopathology 2009;54:355-64.

[2] Bell SW, Kempson RL, Hendrickson MR. Problematic uterine smooth muscle neoplasms. A clinicopathologic study of 213 cases. Am J Surg Pathol 1994;18:535-58.

[3] Oliva E, Carcangiu ML, Carinelli SG, et al. Mesenchymal tumours. Smooth muscle tumour of uncertain malignant potential. In: Kurman RJ, Carcangiu ML, Herrington CS, Young RH, editors. WHO Classification of Tumours of Female Reproductive Organs.Lyon: IARC; 2014. p. 135-47.

[4] de Vos S, Wilczynski SP, Fleischhacker M, Koeffler P. p53 alterations in uterine leiomyosarcomas versus leiomyomas. Gynecol Oncol 1994;54:205-8.

[5] Hall KL, Teneriello MG, Taylor RR, et al. Analysis of Ki-ras, p53, and MDM2 genes in uterine leiomyomas and leiomyosarcomas. Gynecol Oncol 1997;65:330-5.

[6] Jeffers MD, Farquharson MA, Richmond JA, McNicol AM. p53 immunoreactivity and mutation of the p53 gene in smooth muscle tumours of the uterine corpus. J Pathol 1995;177:65-70.

[7] Mayerhofer K, Lozanov P, Bodner K, Bodner-Adler B, Kimberger O, Czerwenka K. Ki-67 expression in patients with uterine leiomyomas, uterine smooth muscle tumors of uncertain malignant potential (STUMP) and uterine leiomyosarcomas (LMS). Acta Obstet Gynecol Scand 2004;83:1085-8.

[8] Niemann TH, Raab SS, Lenel JC, Rodgers JR, Robinson RA. p53 protein overexpression in smooth muscle tumors of the uterus. Hum Pathol 1995;26:375-9.

[9] O'Neill CJ, McBride HA, Connolly LE, McCluggage WG. Uterine leiomyosarcomas are characterized by high p16, p53 and MIB1 expression in comparison with usual leiomyomas, leiomyoma variants and smooth muscle tumours of uncertain malignant potential. Histopathology 2007;50:851-8.

[10] Ip PP, Cheung AN, Clement PB. Uterine smooth muscle tumors of uncertain malignant potential (STUMP): a clinicopathologic analysis of 16 cases. Am J Surg Pathol 2009;33:992-1005.

[11] Ip PP, Tse KY, Tam KF. Uterine smooth muscle tumors other than the ordinary leiomyomas and leiomyosarcomas: a review of selected variants with emphasis on recent advances and unusual morphology that may cause concern for malignancy. Adv Anat Pathol 2010; 17:91-112.

[12] Ip PP, Cheung AN. Pathology of uterine leiomyosarcomas and smooth muscle tumours of uncertain malignant potential. Best Pract Res Clin Obstet Gynaecol 2011;25:691-704.

[13] Cho YL, Bae S, Koo MS, et al. Array comparative genomic hybridization analysis of uterine leiomyosarcoma. Gynecol Oncol 2005;99:545-51.

[14] Meadows KL, Andrews DM, Xu Z, et al. Genome-wide analysis of loss of heterozygosity and copy number amplification in uterine leiomyomas using the 100K single nucleotide polymorphism array. Exp Mol Pathol 2011;91:434-9.

[15] Raish M, Khurshid M, Ansari MA, et al. Analysis of molecular cytogenetic alterations in uterine leiomyosarcoma by array-based comparative genomic hybridization. J Cancer Res Clin Oncol 2012;138:1173-86.

[16] Chibon F, Lagarde P, Salas S, et al. Validated prediction of clinical outcome in sarcomas and multiple types of cancer on the basis of a gene expression signature related to genome complexity. Nat Med 2010;16:781-7.

[17] Lagarde P, Perot G, Kauffmann A, et al. Mitotic checkpoints and chromosome instability are strong predictors of clinical outcome in gastrointestinal stromal tumors. Clin Cancer Res 2012;18:826-38.

[18] Amant F, Moerman P, Vergote I. Report of an unusual problematic uterine smooth muscle neoplasm, emphasizing the prognostic importance of coagulative tumor cell necrosis. Int J Gynecol Cancer 2005;15:1210-2.

[19] D'Angelo E, Espinosa I, Ali R, et al. Uterine leiomyosarcomas: tumor size, mitotic index, and biomarkers Ki67, and Bcl-2 identify two groups with different prognosis. GynecolOnco12011;121:328-33.

[20] Veras E, Zivanovic O, Jacks L, Chiappetta D, Hensley M, Soslow R. "Low-grade leiomyosarcoma." and late-recurring smooth muscle tumors of the uterus: a heterogenous collection of frequently misdiagnosed tumors associated with an overall favorable prognosis relative to conventional uterine leiomyosarcomas. Am J Surg Pathol 2011;35:1626-37.

[21] Lim D, Alvarez T, Nucci MR, et al. Interobserver variability in the interpretation of tumor cell necrosis in uterine leiomyosarcoma. Am J Surg Pathol 2013;37:650-8.

[22] Croce S., Young R.H, Oliva E. Uterine Leiomyomas with Bizarre Nuclei: A Clinicopathologic Study of 59 cases. Am J Surg Pathol 2014.

[23] WO/2010/122243.

[24] WO2012/52954.

[25] Kaplan, E. L.; Meier, P.: Nonparametric estimation from incomplete observations. J. Amer. Statist. Assn. 53:457-481, 1958 Kaplan, E. L.; Meier, P.: Nonparametric estimation from incomplete observations. J. Amer. Statist. Assn. 53:457-481, 1958.

**Claims**

1. Method for *in vitro* classify and/or distinguish, in a patient-derived biological sample of STUMP, truly malignant tumors from benign lesions within a biological sample belonging to the category of STUMP, especially those with equivocal morphological features, **characterized in that** it comprises the calculation of the Genomic Index (GI), i.e. the number and type of alterations of the GIST genome, according to the formula as follows:

$$GI = A^2 / C,$$

wherein A is the total number of alterations in STUMP genome, consisting of segmental gains and losses, and C is the number of involved chromosomes,
and wherein said STUMP is classified in a group without recurrence or metastasis when the GI is less than 10, or in a group of tumors with significant recurrence when GI is equal or higher than 10.

2. Method according to claim 1, wherein the group without recurrence or metastasis has a similar behavior to that of leiomyoma.

3. Method according to claim 1, wherein, in the group of tumors with significant recurrence, said recurrence is due to disease that encompasses a spectrum of smooth muscle lesions approaching the recurrence risk level of leiomyosarcomas but a more indolent, less aggressive course compared to leiomyosarcomas.

4. Method according to any one of the preceding claims, wherein the total number of alterations and the number of involved chromosomes in STUMP in the sample are determined by a genomic analysis by Array comparative genomic hybridization.

5. Method for in vitro predicting survival and/or clinical outcome of a biological sample belonging to the category of STUMP, **characterized in that** it comprises the calculation of the Genomic Index (GI), i.e. the number and type of alterations of the STUMP genome, according to the formula as follows:

$$GI = A^2 / C,$$

wherein A is the total number of alterations, consisting of segmental gains and losses, and C is the number of involved chromosomes in STUMP in the sample,
and wherein said STUMP is classified in a group with low risk to develop metastases within 5 years, i.e. with a risk to develop metastases within 5 years equal to 0%, when GI is less than 10.

**6.** Method according to claim 5, wherein STUMP is classified in a group with high risk to develop metastases within 5 years, i.e. with a risk to develop metastases within 5 years more than 75%, when GI is equal or more than 10.

**7.** Kit for the *in vitro* prediction of the survival outcome of a patient suffering from STUMP or from a Smooth muscle uterine tumor, and/or the development of metastases in a patient treated for or suffering from STUMP or from a Smooth muscle uterine tumor , and/or the prediction of the efficacy of a treatment for STUMP or from a Smooth muscle uterine tumor, **characterized in that** it comprises means for the calculation of the GI.

**8.** Method for screening for compounds for the use in the treatment of STUMP, **characterized in that** it comprises the steps of:

  a. Contacting a test compound with a patient-derived biological sample containing STUMP cells,
  b. Calculating GI,
  c. Selecting said test compound allowing a down-regulation of the GI to 10 or less;

wherein the STUMP is a leiomyosarcoma.

**9.** Method of follow-up of efficacy of a treatment of STUMP by a compound, **characterized in that** it comprises the steps of :

  a. Calculating GI in a patient-derived biological sample containing STUMP cells before the beginning of the treatment of the patient by the compound,
  b. Calculating GI in a patient-derived biological sample containing STUMP cells during the treatment of the patient by the compound,
  c. Comparing said GI of step a) and said GI of step b),

wherein the decrease of the GI calculated in step b) indicates an efficient treatment of STUMP.

**Figure 1** (1/2)

**Figure 1** (2/2)

A

B

# Figure 2

**A**

**B**

# Figure 3

**A**

STUMP - Genomic Index

|  |  | 0 | 1 y | 2 y | 3 y | 4 y | 5 y |
|---|---|---|---|---|---|---|---|
|  | Patients at risk | 15 | 14 | 14 | 11 | 11 | 10 |
| GI<10 | Cumulated events | 0 | 0 | 0 | 0 | 0 | 0 |
|  | Metastasis FS | 1 | 1 | 1 | 1 | 1 | 1 |
|  | Patients at risk | 13 | 11 | 8 | 7 | 4 | 2 |
| GI>10 | Cumulated events | 0 | 2 | 4 | 5 | 6 | 8 |
|  | Metastasis FS | 1 | 0.85 | 0.69 | 0.61 | 0.52 | 0.26 |

# Figure 4 (1/3)

B

**STUMP / LM / LMS- Genomic Index**

a : STUMP GI<10 : 15 cases

b : STUMP GI>10 : 13 cases

c: LM: 8 cases

d: LMS: 8 cases

|  |  | 0 | 1 y | 2 y | 3 y | 4 y | 5 y |
|---|---|---|---|---|---|---|---|
| STUMP GI<10 | Patients at risk | 15 | 14 | 14 | 11 | 11 | 10 |
|  | Cumulated events | 0 | 0 | 0 | 0 | 0 | 0 |
|  | Metastasis FS | 1 | 1 | 1 | 1 | 1 | 1 |
| STUMP GI>10 | Patients at risk | 13 | 11 | 8 | 7 | 4 | 2 |
|  | Cumulated events | 0 | 2 | 4 | 5 | 6 | 8 |
|  | Metastasis FS | 1 | 0.85 | 0.69 | 0.61 | 0.52 | 0.26 |
| LM | Patients at risk | 8 | 6 | 5 | 2 |  |  |
|  | Cumulated events | 0 | 0 | 0 | 0 |  |  |
|  | Metastasis FS | 1 | 1 | 1 | 1 |  |  |
| LMS | Patients at risk | 8 | 1 | 1 | 1 |  |  |
|  | Cumulated events | 0 | 4 | 4 | 4 |  |  |
|  | Metastasis FS | 1 | 0.23 | 0.23 | 0.23 |  |  |

**Figure 4 (2/3)**

C

## STUMP / LM / LMS- Genomic Index

a : STUMP GI<10 : 15 cases

b : STUMP GI>10 : 13 cases

c:  LM: 8 cases

d: LMS: 8 cases

| | | 0 | 1 y | 2 y | 3 y | 4 y | 5 y |
|---|---|---|---|---|---|---|---|
| STUMP GI<10 | Patients at risk | 15 | 14 | 14 | 11 | 11 | 10 |
| | Cumulated events | 0 | 0 | 0 | 0 | 0 | 0 |
| | Overall S | 1 | 1 | 1 | 1 | 1 | 1 |
| STUMP GI>10 | Patients at risk | 13 | 13 | 11 | 9 | 7 | 6 |
| | Cumulated events | 0 | 0 | 1 | 2 | 2 | 2 |
| | Overall S | 1 | 1 | 0.92 | 0.83 | 0.83 | 0.83 |
| LM | Patients at risk | 8 | 6 | 5 | 2 | | |
| | Cumulated events | 0 | 0 | 0 | 0 | | |
| | Overall S | 1 | 1 | 1 | 1 | | |
| LMS | Patients at risk | 8 | 4 | 3 | 2 | 1 | |
| | Cumulated events | 0 | 4 | 5 | 5 | 6 | |
| | Overall S | 1 | 0.50 | 0.37 | 0.37 | 0.18 | |

## Figure 4 (3/3)

Europäisches
Patentamt

European
Patent Office

Office européen
des brevets

**EUROPEAN SEARCH REPORT**

Application Number

EP 15 30 5347

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2012/052954 A1 (UNIV BORDEAUX SEGALEN [FR]; INST NAT SANTE RECH MED [FR]; INST BERGONI) 26 April 2012 (2012-04-26) <br> * claims 1-11 * <br> * page 4, paragraph 3 - page 7 * <br> * page 59, paragraph 2 * | 1-8 | INV. <br> C12Q1/68 |
| X | P. LAGARDE ET AL: "Mitotic Checkpoints and Chromosome Instability Are Strong Predictors of Clinical Outcome in Gastrointestinal Stromal Tumors", CLINICAL CANCER RESEARCH, vol. 18, no. 3, 13 December 2011 (2011-12-13), pages 826-838, XP055210417, ISSN: 1078-0432, DOI: 10.1158/1078-0432.CCR-11-1610 | 7 | |
| Y | * the whole document * <br> * abstract * <br> * page 827, column 2, paragraphs 2,4 * <br> * page 829, column 2, last paragraph - page 836, column 2 * | 1-6,8 | |
| X | LYDIA LARTIGUE ET AL: "Genomic index predicts clinical outcome of intermediate-risk gastrointestinal stromal tumours, providing a new inclusion criterion for imatinib adjuvant therapy", EUROPEAN JOURNAL OF CANCER, vol. 51, no. 1, 6 November 2014 (2014-11-06), pages 75-83, XP055210440, ISSN: 0959-8049, DOI: 10.1016/j.ejca.2014.10.014 | 7 | TECHNICAL FIELDS SEARCHED (IPC) <br><br> C12Q |
| Y | * the whole document * <br> * paragraphs [02.2], [0004] * <br> * figure 4 * | 1-6,8 | |

-/--

The present search report has been drawn up for all claims

| Place of search | Date of completion of the search | Examiner |
|---|---|---|
| The Hague | 2 September 2015 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 30 5347

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PHILIP P C IP ET AL: "Pathology of uterine leiomyosarcomas and smooth muscle tumours of uncertain malignant potential", BAILLIERE'S BEST PRACTICE AND RESEARCH. CLINICAL OBSTETRICS ANDGYNAECOLOGY, vol. 25, no. 6, 21 July 2011 (2011-07-21), pages 691-704, XP028326308, ISSN: 1521-6934, DOI: 10.1016/J.BPOBGYN.2011.07.003 [retrieved on 2011-07-21] * the whole document * | 1-6,8 | |
| Y | Klaus Mayerhofer ET AL: "Ki-67 expression in patients with uterineleiomyomas, uterine smooth muscle tumorsof uncertain malignant potential (STUMP)and uterine leiomyosarcomas (LMS)", acta obstet gynecol scand, vol. 83 1 November 2004 (2004-11-01), 1 November 2004 (2004-11-01), pages 1085-1088, XP055210706, Retrieved from the Internet: URL:http://www.sciencedirect.com/science/article/pii/S0090825899954367 [retrieved on 2015-09-02] * the whole document * | 1-6,8 | |
| A | PRZYBYL JOANNA ET AL: "Metastatic potential is determined early in synovial sarcoma development and reflected by tumor molecular features", INTERNATIONAL JOURNAL OF BIOCHEMISTRY AND CELL BIOLOGY, PERGAMON, GB, vol. 53, 16 May 2014 (2014-05-16), pages 505-513, XP029040293, ISSN: 1357-2725, DOI: 10.1016/J.BIOCEL.2014.05.006 * the whole document * | 1-9 | |

TECHNICAL FIELDS
SEARCHED      (IPC)

The present search report has been drawn up for all claims

| Place of search | Date of completion of the search | Examiner |
|---|---|---|
| The Hague | 2 September 2015 | Bruma, Anja |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 15 30 5347

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2015

| Patent document cited in search report | Publication date | Patent family member(s) | Publication date |
|---|---|---|---|
| WO 2012052954 A1 | 26-04-2012 | CA 2811744 A1 | 26-04-2012 |
| | | DK 2630259 T3 | 06-07-2015 |
| | | EP 2630259 A1 | 28-08-2013 |
| | | ES 2541054 T3 | 15-07-2015 |
| | | JP 2014501496 A | 23-01-2014 |
| | | US 2013288250 A1 | 31-10-2013 |
| | | WO 2012052954 A1 | 26-04-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010122243 A

- WO 201252954 A

### Non-patent literature cited in the description

- **ABELER VM et al.** Uterine sarcomas in Norway. A histopathological and prognostic survey of a total population from 1970 to 2000 including 419 patients. *Histopathology,* 2009, vol. 54, 355-64
- **BELL SW et al.** Problematic uterine smooth muscle neoplasms. A clinicopathologic study of 213 cases. *Am J Surg Pathol,* 1994, vol. 18, 535-58
- Mesenchymal tumours. Smooth muscle tumour of uncertain malignant potential. **OLIVA E et al.** WHO Classification of Tumours of Female Reproductive Organs.Lyon: IARC. 2014, 135-47
- **AMANT F et al.** Report of an unusual problematic uterine smooth muscle neoplasm, emphasizing the prognostic importance of coagulative tumor cell necrosis. *Int J Gynecol Cancer,* 2005, vol. 15, 1210-2
- **D'ANGELO E et al.** Uterine leiomyosarcomas: tumor size, mitotic index, and biomarkers Ki67, and Bcl-2 identify two groups with different prognosis. *Gynecol Oncol,* 2011, vol. 121, 328-33
- **VERAS E et al.** Low-grade leiomyosarcoma" and late-recurring smooth muscle tumors of the uterus: a heterogenous collection of frequently misdiagnosed tumors associated with an overall favorable prognosis relative to conventional uterine leiomyosarcomas. *Am J Surg Pathol,* 2011, vol. 35, 1626-37
- **LIM D et al.** Interobserver variability in the interpretation of tumor cell necrosis in uterine leiomyosarcoma. *Am J Surg Pathol,* 2013, vol. 37, 650-8
- **DE VOS S et al.** p53 alterations in uterine leiomyosarcomas versus leiomyomas. *Gynecol Oncol,* 1994, vol. 54, 205-8
- **HALL KL et al.** Analysis of Ki-ras, p53, and MDM2 genes in uterine leiomyomas and leiomyosarcomas. *Gynecol Oncol,* 1997, vol. 65, 330-5
- **JEFFERS MD et al.** p53 immunoreactivity and mutation of the p53 gene in smooth muscle tumours of the uterine corpus. *J Pathol,* 1995, vol. 177, 65-70
- **MAYERHOFER K et al.** Ki-67 expression in patients with uterine leiomyomas, uterine smooth muscle tumors of uncertain malignant potential (STUMP) and uterine leiomyosarcomas (LMS). *Acta Obstet Gynecol Scand,* 2004, vol. 83, 1085-8

- **NIEMANN TH et al.** p53 protein overexpression in smooth muscle tumors of the uterus. *Hum Pathol,* 1995, vol. 26, 375-9
- **O'NEILL CJ et al.** Uterine leiomyosarcomas are characterized by high p16, p53 and MIB1 expression in comparison with usual leiomyomas, leiomyoma variants and smooth muscle tumours of uncertain malignant potential. *Histopathology,* 2007, vol. 50, 851-8
- **IP PP et al.** Uterine smooth muscle tumors of uncertain malignant potential (STUMP): a clinicopathologic analysis of 16 cases. *Am J Surg Pathol,* 2009, vol. 33, 992-1005
- **IP PP et al.** Uterine smooth muscle tumors other than the ordinary leiomyomas and leiomyosarcomas: a review of selected variants with emphasis on recent advances and unusual morphology that may cause concern for malignancy. *Adv Anat Pathol,* 2010, vol. 17, 91-11210
- **IP PP, CHEUNG AN.** Pathology of uterine leiomyosarcomas and smooth muscle tumours of uncertain malignant potential. *Best Pract Res Clin Obstet Gynaecol,* 2011, vol. 25, 691-704
- **CHO YL ; BAE S ; KOO MS et al.** Array comparative genomic hybridization analysis of uterine leiomyosarcoma. *Gynecol Oncol,* 2005, vol. 99, 545-51
- **MEADOWS KL et al.** Genome-wide analysis of loss of heterozygosity and copy number amplification in uterine leiomyomas using the 100K single nucleotide polymorphism array. *Exp Mol Pathol,* 2011, vol. 91, 434-9
- **RAISH M ; KHURSHID M ; ANSARI MA et al.** Analysis of molecular cytogenetic alterations in uterine leiomyosarcoma by array-based comparative genomic hybridization. *J Cancer Res Clin Oncol,* 2012, vol. 138, 1173-86
- **CHIBON F et al.** Validated prediction of clinical outcome in sarcomas and multiple types of cancer on the basis of a gene expression signature related to genome complexity. *Nat Med,* 2010, vol. 16, 781-7

- **CROCE S. et al.** Uterine Leiomyomas with Bizarre Nuclei: A Clinicopathologic Study of 59 cases. *Am J Surg Pathol,* 2014
- **LAGARDE P et al.** Mitotic checkpoints and chromosome instability are strong predictors of clinical outcome in gastrointestinal stromal tumors. *Clin Cancer Res,* 2012, vol. 18, 826-38
- **KAPLAN, E. L. ; MEIER, P.** Nonparametric estimation from incomplete observations. *J. Amer. Statist. Assn.,* 1958, vol. 53, 457-481
- **ABELER VM ; ROYNE O ; THORESEN S ; DANIELSEN HE ; NESLAND JM ; KRISTENSEN GB.** Uterine sarcomas in Norway. A histopathological and prognostic survey of a total population from 1970 to 2000 including 419 patients. *Histopathology,* 2009, vol. 54, 355-64
- **BELL SW ; KEMPSON RL ; HENDRICKSON MR.** Problematic uterine smooth muscle neoplasms. A clinicopathologic study of 213 cases. *Am J Surg Pathol,* 1994, vol. 18, 535-58
- Mesenchymal tumours. Smooth muscle tumour of uncertain malignant potential. **OLIVA E ; CARCANGIU ML ; CARINELLI SG et al.** WHO Classification of Tumours of Female Reproductive Organs.Lyon: IARC. 2014, 135-47
- **DE VOS S ; WILCZYNSKI SP ; FLEISCHHACKER M ; KOEFFLER P.** p53 alterations in uterine leiomyosarcomas versus leiomyomas. *Gynecol Oncol,* 1994, vol. 54, 205-8
- **HALL KL ; TENERIELLO MG ; TAYLOR RR et al.** Analysis of Ki-ras, p53, and MDM2 genes in uterine leiomyomas and leiomyosarcomas. *Gynecol Oncol,* 1997, vol. 65, 330-5
- **JEFFERS MD ; FARQUHARSON MA ; RICHMOND JA ; MCNICOL AM.** p53 immunoreactivity and mutation of the p53 gene in smooth muscle tumours of the uterine corpus. *J Pathol,* 1995, vol. 177, 65-70
- **MAYERHOFER K ; LOZANOV P ; BODNER K ; BODNER-ADLER B ; KIMBERGER O.** Czerwenka K. Ki-67 expression in patients with uterine leiomyomas, uterine smooth muscle tumors of uncertain malignant potential (STUMP) and uterine leiomyosarcomas (LMS). *Acta Obstet Gynecol Scand,* 2004, vol. 83, 1085-8
- **NIEMANN TH ; RAAB SS ; LENEL JC ; RODGERS JR ; ROBINSON RA.** p53 protein overexpression in smooth muscle tumors of the uterus. *Hum Pathol,* 1995, vol. 26, 375-9
- **O'NEILL CJ ; MCBRIDE HA ; CONNOLLY LE ; MCCLUGGAGE WG.** Uterine leiomyosarcomas are characterized by high p16, p53 and MIB1 expression in comparison with usual leiomyomas, leiomyoma variants and smooth muscle tumours of uncertain malignant potential. *Histopathology,* 2007, vol. 50, 851-8
- **IP PP ; CHEUNG AN ; CLEMENT PB.** Uterine smooth muscle tumors of uncertain malignant potential (STUMP): a clinicopathologic analysis of 16 cases. *Am J Surg Pathol,* 2009, vol. 33, 992-1005
- **IP PP ; TSE KY ; TAM KF.** Uterine smooth muscle tumors other than the ordinary leiomyomas and leiomyosarcomas: a review of selected variants with emphasis on recent advances and unusual morphology that may cause concern for malignancy. *Adv Anat Pathol,* 2010, vol. 17, 91-112
- **IP PP ; CHEUNG AN.** Pathology of uterine leiomyosarcomas and smooth muscle tumours of uncertain malignant potential. *Best Pract Res Clin Obstet Gynaecol,* 2011, vol. 25, 691-704
- **MEADOWS KL ; ANDREWS DM ; XU Z et al.** Genome-wide analysis of loss of heterozygosity and copy number amplification in uterine leiomyomas using the 100K single nucleotide polymorphism array. *Exp Mol Pathol,* 2011, vol. 91, 434-9
- **CHIBON F ; LAGARDE P ; SALAS S et al.** Validated prediction of clinical outcome in sarcomas and multiple types of cancer on the basis of a gene expression signature related to genome complexity. *Nat Med,* 2010, vol. 16, 781-7
- **LAGARDE P ; PEROT G ; KAUFFMANN A et al.** Mitotic checkpoints and chromosome instability are strong predictors of clinical outcome in gastrointestinal stromal tumors. *Clin Cancer Res,* 2012, vol. 18, 826-38
- **AMANT F ; MOERMAN P ; VERGOTE I.** Report of an unusual problematic uterine smooth muscle neoplasm, emphasizing the prognostic importance of coagulative tumor cell necrosis. *Int J Gynecol Cancer,* 2005, vol. 15, 1210-2
- **D'ANGELO E ; ESPINOSA I ; ALI R et al.** Uterine leiomyosarcomas: tumor size, mitotic index, and biomarkers Ki67, and Bcl-2 identify two groups with different prognosis. *GynecolOnco1,* 2011, vol. 121, 328-33
- **VERAS E ; ZIVANOVIC O ; JACKS L ; CHIAPPETTA D ; HENSLEY M ; SOSLOW R.** Low-grade leiomyosarcoma." and late-recurring smooth muscle tumors of the uterus: a heterogenous collection of frequently misdiagnosed tumors associated with an overall favorable prognosis relative to conventional uterine leiomyosarcomas. *Am J Surg Pathol,* 2011, vol. 35, 1626-37
- **LIM D ; ALVAREZ T ; NUCCI MR et al.** Interobserver variability in the interpretation of tumor cell necrosis in uterine leiomyosarcoma. *Am J Surg Pathol,* 2013, vol. 37, 650-8
- **CROCE S. ; YOUNG R.H ; OLIVA E.** Uterine Leiomyomas with Bizarre Nuclei: A Clinicopathologic Study of 59 cases. *Am J Surg Pathol,* 2014